# EUROPEAN PATENT APPLICATION

(11) **EP 4 194 005 A1**
(43) Date of publication of application: **14.06.2023**
(21) Application number: 21861702.5
(22) Date of filing: 27.08.2021
(51) Int. Cl.: A61K 38/42, A61K 9/127, A61K 9/48, A61K 35/18, A61P 39/02

(54) **MEDICINE CONTAINING METHEMOGLOBIN ENDOPLASMIC RETICULUM AS ACTIVE INGREDIENT AND USE THEREOF**

(30) Priority: 28.08.2020 JP 2020144044
(71) Applicant: Keio University, Tokyo, 108-8345 (JP); Public University Corporation Nara Medical University, Nara 634-8521 (JP); Kimigafuchi Gakuen, Kumamoto-shi, Kumamoto 860-0082 (JP)
(72) Inventor: TAGUCHI Kazuaki, Tokyo 105-8512 (JP); SUZUKI Yuto, Tokyo 105-8512 (JP); MATSUMOTO Kazuaki, Tokyo 105-8512 (JP); SAKAI Hiromi, Kashihara-shi, Nara 634-8521 (JP); OTAGIRI Masaki, Kumamoto-shi, Kumamoto 860-0082 (JP)
(74) Representative: Isarpatent
(86) International application number: PCT/JP2021/031458
(87) International publication number: WO 2022/045281

(57) **Abstract**

The present invention is intended to provide a medicament for cyanide poisoning, hydrogen sulfide poisoning, and azide poisoning, wherein the medicament does not cause side effects, is excellent in immediate effectiveness, and has a high antidotal effect. Specifically, the present invention relates to a medicament for cyanide poisoning, hydrogen sulfide poisoning, and azide poisoning, wherein the medicament comprises, as an active ingredient(s), one or more selected from the group consisting of a heme protein capable of binding to cyanide ions (CN⁻), hydrogen sulfide ions (HS⁻) and azide ions (N₃⁻), a substance containing a heme protein capable of binding to CN⁻, HS⁻ and N₃⁻, and a heme derivative capable of binding to CN⁻, HS⁻ and N₃⁻. More specifically, the present invention relates to a medicament or a pharmaceutical composition for the treatment or prophylaxis of cyanide poisoning, hydrogen sulfide poisoning, and azide poisoning.

## Description

### Technical Field

The present invention relates to a medicament for the treatment or prophylaxis of cyanide poisoning, hydrogen sulfide poisoning, and azide poisoning.

### Background Art

Cyanide poisoning is caused by a phenomenon that cyanide ions (CN⁻) bind to cytochrome c oxidase (CcOX) in intracellular mitochondria and directly inhibit the oxygen metabolism of cells. There are a wide variety of factors that cause such cyanide poisoning. For example, the cyanide poisoning is caused by a damage associated with the purpose of using cyanide for suicide or terrorism, and further, by unintentional inhalation of cyanide due to combustion of synthetic fibers by fire. The symptoms of cyanide poisoning progress quickly, and the tissues in the whole body become asphyxiated. Thus, the treatment therefor must be initiated as soon as possible.

Hydrogen sulfide (H₂S) is a substance having high water solubility, and at physiological pH, about two-thirds of the H₂S is present in the form of hydrogen sulfide ions (HS⁻) and about one-thirds thereof is present in the form of undissociated H₂S. Such hydrogen sulfide is always present in a trace amount in a living body, and plays a role for regulation of vascular functions, protection of cells, and the like. Hence, the hydrogen sulfide is positioned as one of physiologically active gases. On the other hand, the hydrogen sulfide has strong toxicity, and if an organism is exposed to an excessive amount of hydrogen sulfide at once, acute poisoning is developed. Statistical annual deaths due to hydrogen sulfide poisoning are unknown, but a large number of workers who work in industrial fields including, as typical examples, agriculture and industries such as petroleum are exposed to hydrogen sulfide. One-thirds of petroleum industry workers have experienced some symptoms due to exposure to hydrogen sulfide, and there are also cases where the workers would lead to unconsciousness. Furthermore, since hydrogen sulfide is easily generated from simple chemical substances, in recent years, hydrogen sulfide has been used for suicidal purpose or as a raw material for weapons of mass destruction used by terrorists, as in the case of cyanide ions.

In addition, sodium azide (NaN₃) is a white solid having high water solubility, and has been used as an antiseptic or as an insecticide having a broad spectrum. On the other hand, azide poisoning is caused by accidental ingestion of sodium azide used as an antiseptic. Moreover, sodium azide has been frequently used as a causative substance of homicides by being deliberately mixed into foods and beverages.

As existing methods for detoxifying cyanide poisoning, two types of therapies, namely, the combined use of nitrous acid-based drugs (amyl nitrite and sodium nitrite, etc.) and sodium thiosulfate, and a single use of hydroxocobalamin, have been mainly adopted. However, under the current circumstance, the lethal rate of cyanide poisoning still remains high, and thus, it cannot be said that the above two therapies are best options for the treatment of the cyanide poisoning. Regarding the combined therapy of nitrous acid-based drugs with sodium thiosulfate, induction of hypoxemia by nitrous acid has caused a serious problem. Such nitrous acid-based drugs convert hemoglobin in erythrocytes to methemoglobin (MetHb) and allow CcOX-bound CN⁻ to transfer to MetHb having higher cyanide affinity, so that the functions of CcOX can be recovered (Non Patent Literature 1). Hence, it is highly likely that the oxygen carrying capacity of hemoglobin in erythrocytes is inhibited and hypoxemia is thereby induced, and thus, it is difficult to use the nitrous acid-based drugs for difficulty in breathing or in the event of a fire involving carbon monoxide (CO) poisoning (Non Patent Literature 2).

In hydroxocobalamin monotherapy, a reduction in antidotal ability is an important issue. Since hydroxocobalamin exhibits antidotal ability as a result of direct coordination of CN⁻, hydroxocobalamin does not induce hypoxemia, differing from nitrous acid-based drugs, and thus, it can be used in the event of a fire. However, it has been reported that hydroxocobalamin binds to plasma proteins such as haptocorrin because it is a low-molecular-weight substance, and thus that the antidotal ability of hydroxocobalamin is significantly reduced (1/600). Hence, it is highly likely that hydroxocobalamin will not function as an antidote having immediate effectiveness in an *in vivo* environment (Non Patent Literature 3).

With regard to the mechanism of development of hydrogen sulfide poisoning and azide poisoning, each substance (hydrogen sulfide or azide) that has entered the body through various routes such as oral, transdermal, mucosal, and inhalation routes binds to CcOX and inhibits oxidative phosphorylation, so as to induce hydrogen sulfide poisoning or azide poisoning. Although both hydrogen sulfide poisoning and azide poisoning are acute toxicity causing a high lethal rate, there have not yet been an antidote for hydrogen sulfide poisoning and an antidote for azide poisoning, which have been approved over the world. Interestingly, the mechanism of development of hydrogen sulfide poisoning and azide poisoning is partially similar to that of cyanide poisoning. For this reason, sodium nitrite and hydroxocobalamin approved as antidotes for cyanide poisoning, or compounds similar thereto, have been studied, in terms of the efficacy thereof as antidotes for hydrogen sulfide poisoning and azide poisoning (Non Patent Literatures 12 to Non Patent Literature 17).

As described above, it is an urgent task to develop a method for preventing and treating cyanide poisoning, hydrogen sulfide poisoning, and azide poisoning, and studies have been vigorously promoted. Under the current circumstances, however, there have not existed prophylactic and therapeutic agents having excellent immediate effectiveness and a high antidotal effect.

### Citation List

### Non Patent Literature

Non Patent Literature 1: Baskin et al., The Journal of Clinical Pharmacology 1992; 32(4): 368-375.
Non Patent Literature 2: Brenner et al., Toxicology and Applied Pharmacology 2010; 248(3): 269-276.
Non Patent Literature 3: Watanabe et al., ACS Medicinal Chemistry Letters 2011; 2(12): 943-947.
Non Patent Literature 4: Taguchi et al., Journal of Pharmaceutical Sciences 2011; 100(2): 775-783.
Non Patent Literature 5: Sakai et al., Journal of Internal Medicine 2007; 263(1): 4-15.
Non Patent Literature 6: Sakai et al., Bioconjugate Chemistry 2014; 25(7): 1301-1310.
Non Patent Literature 7: Kettisen et al., Bioconjugate Chemistry 2015; 26(4): 746-754.
Non Patent Literature 8: Cabrales et al., Resuscitation 2007; 75(1): 124-134.
Non Patent Literature 9: Taguchi et al., Drug Metabolism and Disposition 2009; 37(7): 1456-1463.
Non Patent Literature 10: Gu et al., Arch Toxicol. 2018; 92(12): 3505-3515.
Non Patent Literature 11: Cambal et al., Chemical Research in Toxicology 2013; 26(5): 828-836.
Non Patent Literature 12: Frawley et al., Chem. Res. Toxicol. 2020; 33: 594-603.
Non Patent Literature 13: Praekunatham et al., Chem. Res. Toxicol. 2020; 33: 333-342.
Non Patent Literature 14: Jiang et al., Sci. Rep. 2016; 6: 1-10.
Non Patent Literature 15: Ng et al., Clin. Toxicol. 2019; 57: 189-196.
Non Patent Literature 16: Haouzi et al., Toxicol. Sci. 2019; 168: 443-459.
Non Patent Literature 17: Truong et al., Toxicology. 2007; 242: 16-22.

### Summary of Invention

### Technical Problem

Considering the aforementioned circumstances, it is an object of the present invention to provide an antidote for cyanide poisoning, hydrogen sulfide poisoning, and azide poisoning, wherein the antidote has excellent immediate effectiveness and a high antidotal effect.

### Solution to Problem

In order to achieve the aforementioned object, the present inventors have studied utilization of a hemoglobin vesicle (HbV) (Non Patent Literature 4 and Non Patent Literature 5) that is an artificial erythrocyte preparation obtained by incorporating a high concentration of human hemoglobin into a liposome for an antidote for cyanide poisoning, hydrogen sulfide poisoning, and azide poisoning.

MetHb generated as a result of oxidation of hemoglobin (Hb) does not have oxygen binding ability and loses the function of carrying oxygen in a living body. However, the present inventors have discovered that MetHb has binding ability to CN⁻, hydrogen sulfide ions (HS⁻), and azide ions (N₃⁻). Specifically, the present inventors have conceived that, by using a methemoglobin vesicle (MetHbV) in which the Hb in HbV is converted to MetHb, the problems of the aforementioned existing antidotes for cyanide poisoning could be solved, and an antidote for cyanide poisoning, having excellent effects, could be developed. Further, the present inventors have conceived that MetHbV could function as an effective antidote also for hydrogen sulfide poisoning and azide poisoning, the pathogenic mechanism of which is similar to that of cyanide poisoning, and the inventors have evaluated the efficacy of MetHbV as a therapeutic and prophylactic agent for hydrogen sulfide and azide poisoning.

The present inventors have administered MetHbV to cyanide poisoning mouse models, and have evaluated the influence of MetHbV upon the survival rate of the mouse models, the recovery time from the poisoning, and the like. As a result, it was found that, under sodium cyanide (NaCN) poisoning, the survival rate was significantly increased in a MetHbV-administered mouse model group, compared with a non-treatment group to which only NaCN had been administered, and that the recovery time from the poisoning was short, compared with other existing drug-administration groups. Further, the present inventors have administered NaCN to mice, to which MetHbV had previously been administered, and have found that death caused by cyanide poisoning can be suppressed.

Furthermore, the present inventors have administered MetHbV to hydrogen sulfide poisoning mouse models and azide poisoning mouse models, before and after the onset of each poisoning. As a result, in both types of poisoning mouse models, the effectiveness of MetHbV by prophylactic administration (pre-treatment) and therapeutic administration (post-treatment) was confirmed, and the present inventors have found that the antidotal effect of MetHbV is higher than those of sodium nitrite and hydroxocobalamin.

That is to say, the present inventors have found for the first time that MetHbV exhibits excellent effects as a therapeutic drug and a prophylactic drug for cyanide poisoning, hydrogen sulfide poisoning, and azide poisoning.

Specifically, the present invention includes the following (1) to (6).
(1) A medicament for cyanide poisoning, hydrogen sulfide poisoning, and azide poisoning, wherein
   the medicament comprises, as an active ingredient(s), one or more selected from the group consisting of a heme protein capable of binding to cyanide ions (CN⁻), hydrogen sulfide ions (HS⁻) and azide ions (N₃⁻), a substance containing a heme protein capable of binding to CN⁻, HS⁻ and N₃⁻, and a heme derivative capable of binding to CN⁻, HS⁻ and N₃⁻.
(2) The medicament according to the above (1), wherein the heme protein capable of binding to CN⁻, HS⁻ and N₃⁻ is methemoglobin.
(3) The medicament according to the above (1) or (2), wherein the substance containing a heme protein capable of binding to CN⁻, HS⁻ and N₃⁻ is a methemoglobin-containing erythrocyte or a methemoglobin-containing capsule.
(4) The medicament according to the above (3), wherein the capsule consists of at least one selected from the group consisting of a liposome, a polymersome, and a polymer thin film.
(5) The medicament according to any one of the above (1) to (4), which is for the treatment of cyanide poisoning, hydrogen sulfide poisoning, and azide poisoning.
(6) The medicament according to any one the above (1) to (4), which is for the prophylaxis of cyanide poisoning, hydrogen sulfide poisoning, and azide poisoning.

It is to be noted that, in the present description, the preposition "to" disposed between numbers indicates a numerical value range including the left and right values thereof.

### Advantageous Effects of Invention

The medicament and the pharmaceutical composition for use in cyanide poisoning, hydrogen sulfide poisoning and azide poisoning according to the present invention (hereinafter also referred to as a "medicament, etc.") have such effects that they have high immediate effectiveness, compared with existing drugs, and they are extremely unlikely to cause side effects found in such existing drugs, such as induction of hypoxemia.

Furthermore, the medicament, etc. according to the present invention exhibit excellent effects, not only as therapeutic drugs, but also as prophylactic drugs.

### Brief Description of Drawings

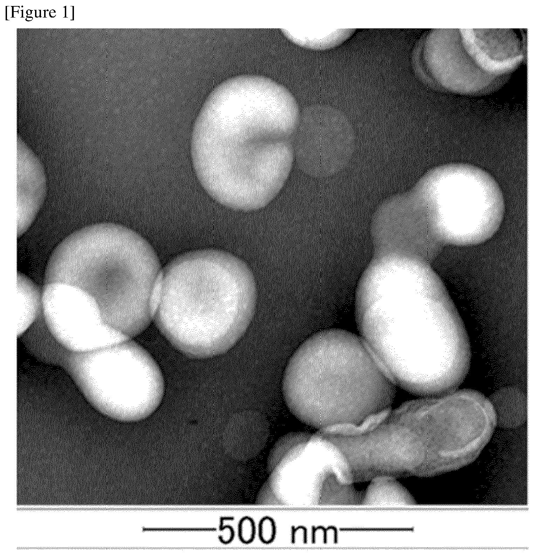

[Figure 1] Figure 1 shows a transmission electron microscope (TEM) image of produced MetHbV.

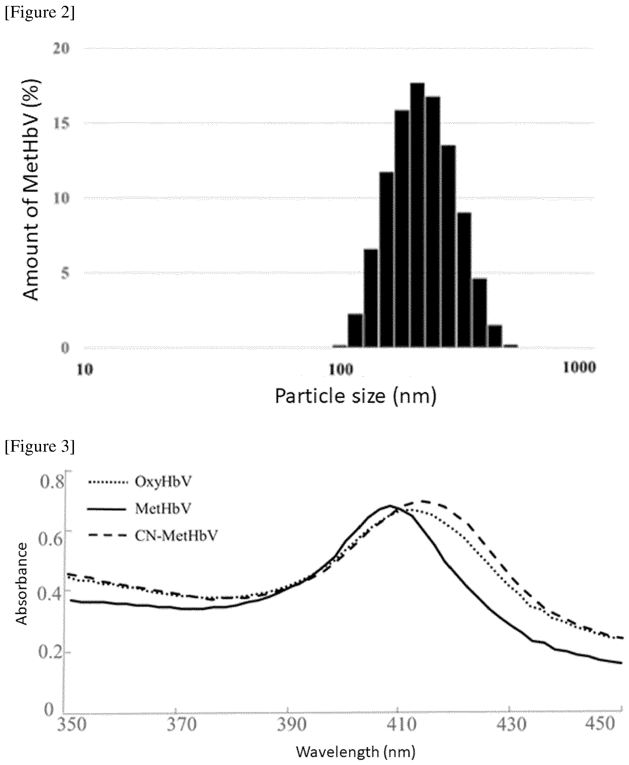

[Figure 2] Figure 2 shows distribution of the particle size of produced MetHbV.
[Figure 3] Figure 3 shows changes of absorption spectra from OxyHbV (Oxyhemoglobin vesicle) to CN⁻MetbV (CN⁻ coordinating MetHbV) through MetHbV. Individual absorption spectra are indicated with the solid line (MetHbV), the dotted line (OxyHbV), and the dashed line (CN⁻MetHbV).

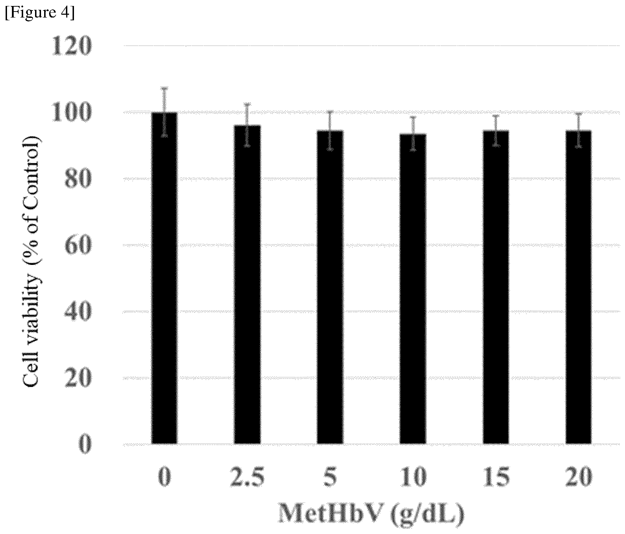

[Figure 4] Figure 4 shows the results obtained by evaluating the cytotoxicity of MetHbV having each different concentration, using CCK-8 (Cell Counting Kit-8). PC-12 cells were used herein. Each value is indicated with a mean value ± standard error (n = 3).

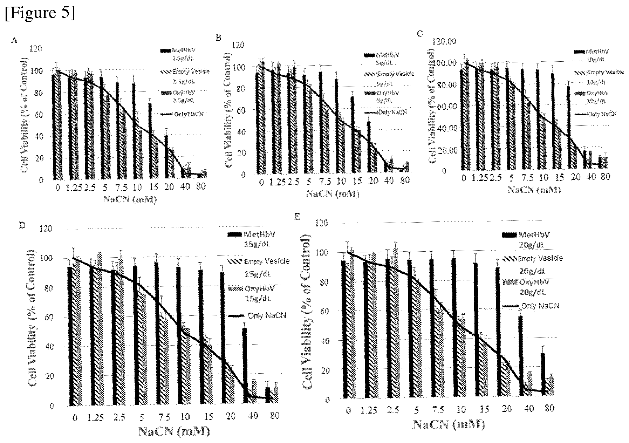

[Figure 5] Figure 5 shows the results obtained by evaluating the effects of the antidotes (MetHbV, OxyHbV, and empty vesicle (EV)) having each different concentration, using CCK-8 (Cell Counting Kit-8). PC-12 cells were used herein. (A) Each antidote was added in an amount of 2.5 g (Hb/dL) or an amount equivalent thereto. (B) Each antidote was added in an amount of 5 g (Hb/dL) or an amount equivalent thereto. (C) Each antidote was added in an amount of 10 g (Hb/dL) or an amount equivalent thereto. (D) Each antidote was added in an amount of 15 g (Hb/dL) or an amount equivalent thereto. (E) Each antidote was added in an amount of 20 g (Hb/dL) or an amount equivalent thereto.

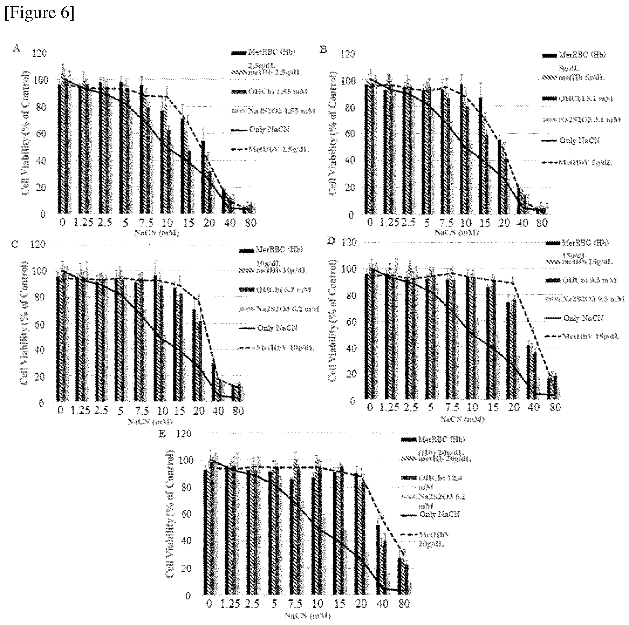

[Figure 6] Figure 6 shows the results obtained by comparing the effects of various antidotes (metRBCs (i.e. red blood cells (RBCs) containing methemoglobin; methemoglobin-containing erythrocyte), metHb (methemoglobin), OHCbl (hydroxocobalamin), and Na₂S₂O₃ (sodium thiosulfate)) with the effects of MetHbV. (A) Each antidote was added in an amount of 2.5 g (Hb/dL) or an amount equivalent thereto. (B) Each antidote was added in an amount of 5 g (Hb/dL) or an amount equivalent thereto. (C) Each antidote was added in an amount of 10 g (Hb/dL) or an amount equivalent thereto. (D) Each antidote was added in an amount of 15 g (Hb/dL) or an amount equivalent thereto. (E) Each antidote was added in an amount of 20 g (Hb/dL) or an amount equivalent thereto.

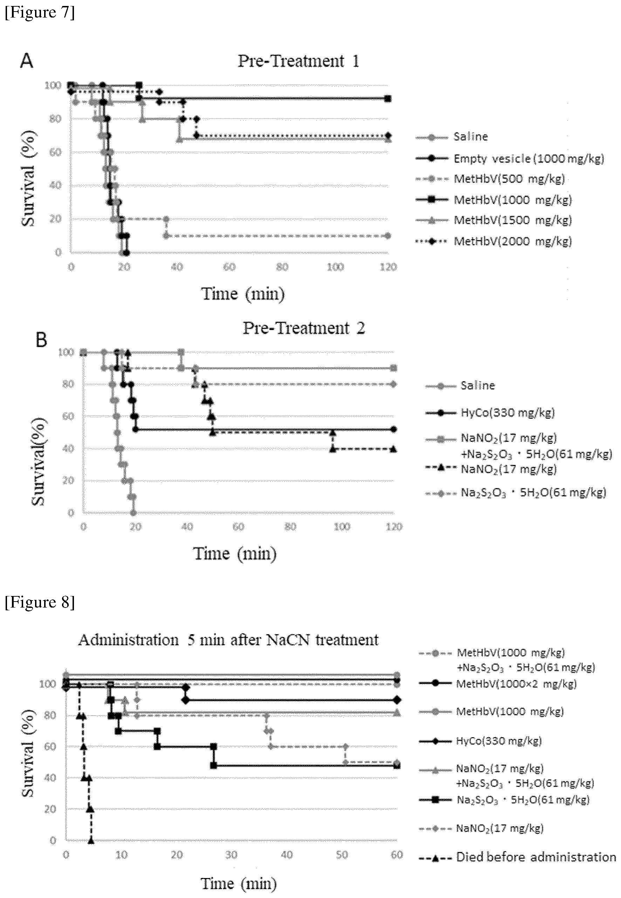

[Figure 7] Figure 7 shows the survival rate of antidote pre-treated mice after administration of NaCN (12 mg/kg, oral administration) once. (A) After MetHbV (500, 1000, 1500 or 2000 mg/kg), an empty vesicle (in an amount equivalent to MetHbV (1000 mg/kg)) or a normal saline (saline) had been intravenously administered to the mice, NaCN was administered thereto. (B) After NaNO₂ (17 mg/kg), Na₂S₂O₃ (61 mg/kg), NaNO₂ (17 mg/kg) and Na₂S₂O₃ (61 mg/kg) (combined administration), OHCbl (330 mg/kg), an empty vesicle (the same as above) or a normal saline had been administered to the mice, NaCN was administered thereto. All of the mice, on which the treatments (A) and (B) had been performed, were observed for 7 days (each mouse group: n = 10).
[Figure 8] Figure 8 shows the survival rate of antidote post-treated mice after administration of NaCN (12 mg/kg, oral administration) once. Five minutes after administration of NaCN to the mice, MetHbV (1000 or 2000 mg/kg), MetHbV (1000 mg/kg) and Na₂S₂O₃ (61 mg/kg) (combined administration), NaNO₂ (17 mg/kg), Na₂S₂O₃ (61 mg/kg), NaNO₂ (17 mg/kg) and Na₂S₂O₃ (61 mg/kg) (combined administration), or OHCbl (330 mg/kg) was intravenously administered to the mice. All of the mice were observed for 7 days (each mouse group: n = 10). Five mice died before administration of the antidote.

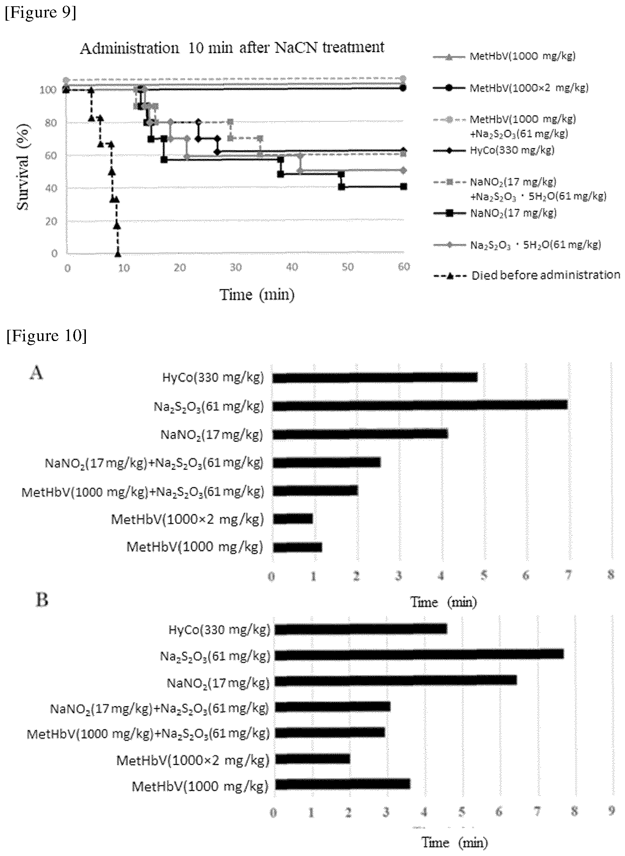

[Figure 9] Figure 9 shows the survival rate of antidote post-treated mice after administration of NaCN (12 mg/kg, oral administration) once. Ten minutes after administration of NaCN to the mice, MetHbV (1000 or 2000 mg/kg), MetHbV (1000 mg/kg) and Na₂S₂O₃ (61 mg/kg) (combined administration), NaNO₂ (17 mg/kg), Na₂S₂O₃ (61 mg/kg), NaNO₂ (17 mg/kg) and Na₂S₂O₃ (61 mg/kg) (combined administration), or OHCbl (330 mg/kg) was intravenously administered to the mice. All of the mice were observed for 7 days (each mouse group: n = 10). Five mice died before administration of the antidote.
[Figure 10] Figure 10 shows that each antidote was administered to NaCN-treated mice and the time required until recovery was then measured. Five minutes (A) or 10 minutes (B) minutes after administration of NaCN to the mice, MetHbV (1000 or 2000 mg/kg), MetHbV (1000 mg/kg) and Na₂S₂O₃ (61 mg/kg) (combined administration), NaNO₂ (17 mg/kg), Na₂S₂O₃ (61 mg/kg), NaNO₂ (17 mg/kg) and Na₂S₂O₃ (61 mg/kg) (combined administration), or OHCbl (330 mg/kg) was intravenously administered to the mice. Thereafter, the time required until awakening was measured. All of the mice were observed from the loss of righting reflex (coma) until awakening.

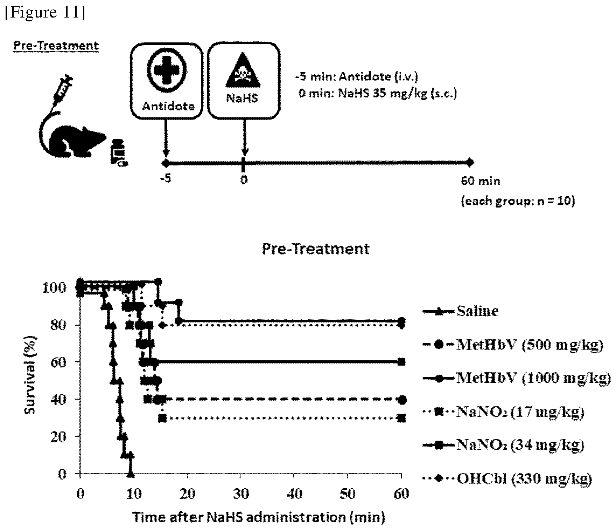

[Figure 11] Figure 11 shows the survival rate of antidote pre-treated mice after administration of NaHS (35 mg/kg, subcutaneous administration) once. The upper view shows an administration schedule of the antidote and NaHS. The lower view shows a change over time in the survival rate of the mice. Five minutes after intravenous administration of the mice with MetHbV (500 or 1000 mg/kg), NaNO₂ (17 or 34 mg/kg), OHCbl (330 mg/kg) or a normal saline, NaHS was administered thereto.

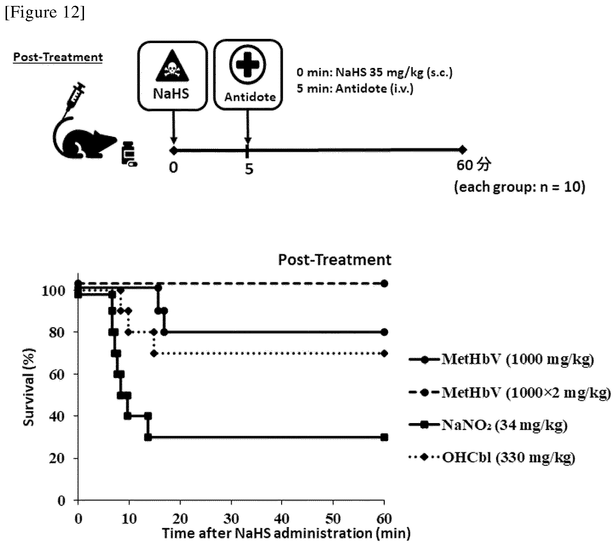

[Figure 12] Figure 12 shows the survival rate of mice, which were post-treated with each antidote after administration of NaHS (35 mg/kg, subcutaneous administration) once. The upper view shows an administration schedule of the antidote and NaHS. The lower view shows a change over time in the survival rate of the mice. Five minutes after administration of the mice with NaHS, MetHbV (1000 or 1000 × 2 mg/kg), NaNO₂ (34 mg/kg), or OHCbl (330 mg/kg) was intravenously administered to the mice.

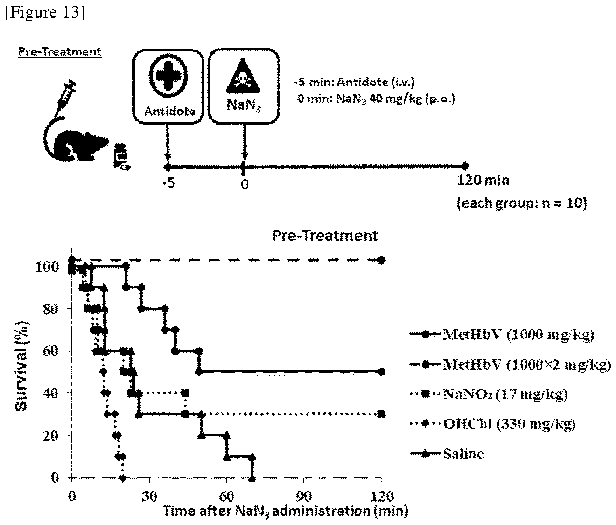

[Figure 13] Figure 13 shows the survival rate of antidote pre-treated mice after administration of NaN₃ (40 mg/kg, oral administration) once. The upper view shows an administration schedule of the antidote and NaN₃. The lower view shows a change over time in the survival rate of the mice. Five minutes after intravenous administration of the mice with MetHbV (1000 or 1000 × 2 mg/kg), NaNO₂ (17 mg/kg), OHCbl (330 mg/kg) or a normal saline, NaHS was administered thereto.

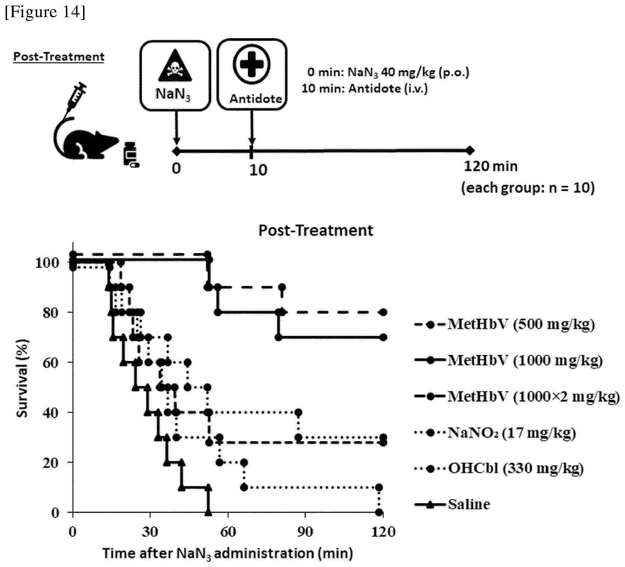

[Figure 14] Figure 14 shows the survival rate of mice, which were post-treated with each antidote after administration of NaN₃ (40 mg/kg, oral administration) once. The upper view shows an administration schedule of the antidote and NaN₃. The lower view shows a change over time in the survival rate of the mice. Ten minutes after administration of the mice with NaHS, MetHbV (500, 1000 or 1000 × 2 mg/kg), NaNO₂ (17 mg/kg), OHCbl (330 mg/kg) or a normal saline was intravenously administered to the mice.

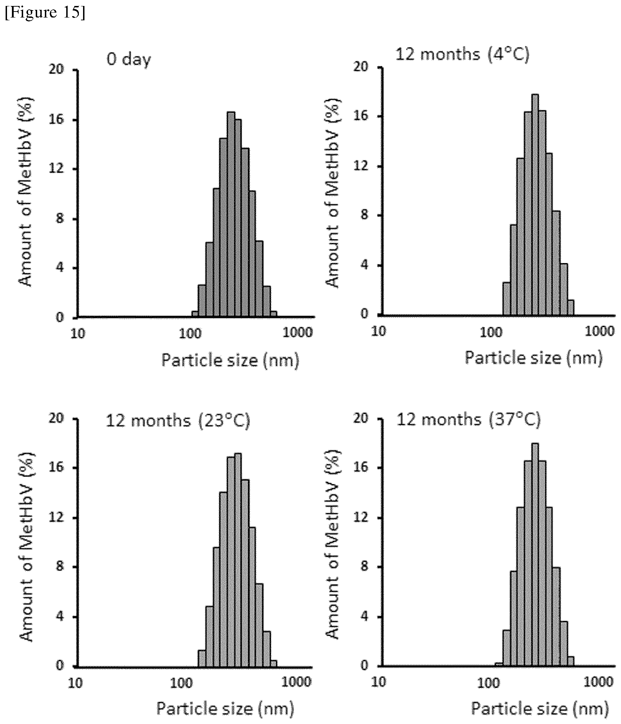

[Figure 15] Figure 15 shows distribution of the particle size of preserved MetHbV. A 10 g Hb/dL MetHbV suspension was preserved in a glass vial under temperature conditions of 4°C, 23°C to 28°C, and 37°C for 1 year. Distribution of the particle size of each preserved MetHbV was evaluated.

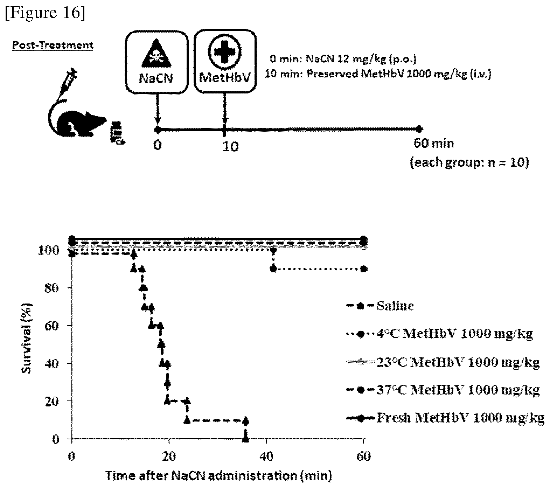

[Figure 16] Figure 16 shows the survival rate of mice, which were post-treated with preserved MetHbV after administration of NaCN (12 mg/kg, oral administration) once. The upper view shows an administration schedule of the preserved MetHbV and NaCN. The lower view shows a change over time in the survival rate of the mice. Ten minutes after administration of NaCN to the mice, MetHbV (Fresh MetHbV) that had not been preserved after preparation, MetHbV (4°C MetHbV) preserved at 4°C for 1 year, MetHbV (23°C MetHbV) preserved at 23°C to 28°C for 1 year, or MetHbV (37°C MetHbV) preserved at 37°C for 1 year was intravenously administered in an amount of 1000 mg/kg each to the mice.

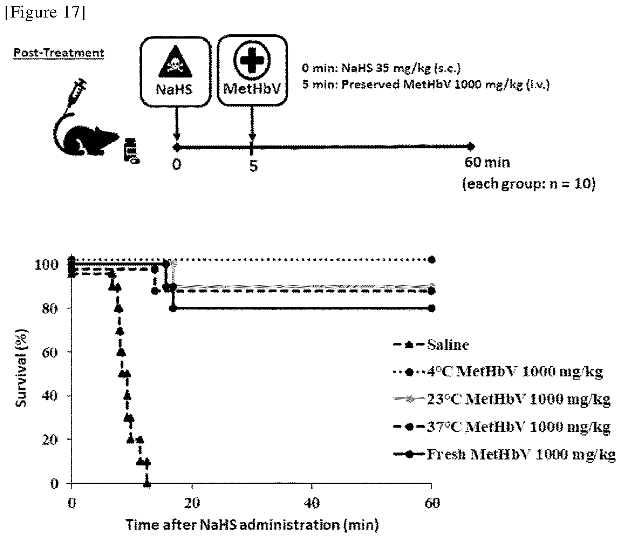

[Figure 17] Figure 17 shows the survival rate of mice, which were post-treated with preserved MetHbV after administration of NaHS (35 mg/kg, subcutaneous administration) once. The upper view shows an administration schedule of the preserved MetHbV and NaHS. The lower view shows a change over time in the survival rate of the mice. Ten minutes after administration of the mice with NaHS, MetHbV (Fresh MetHbV) that had not been preserved after preparation, MetHbV (4°C MetHbV) preserved at 4°C for 1 year, MetHbV (23°C MetHbV) preserved at 23°C to 28°C for 1 year, or MetHbV (37°C MetHbV) preserved at 37°C for 1 year was intravenously administered in an amount of 1000 mg/kg each to the mice.

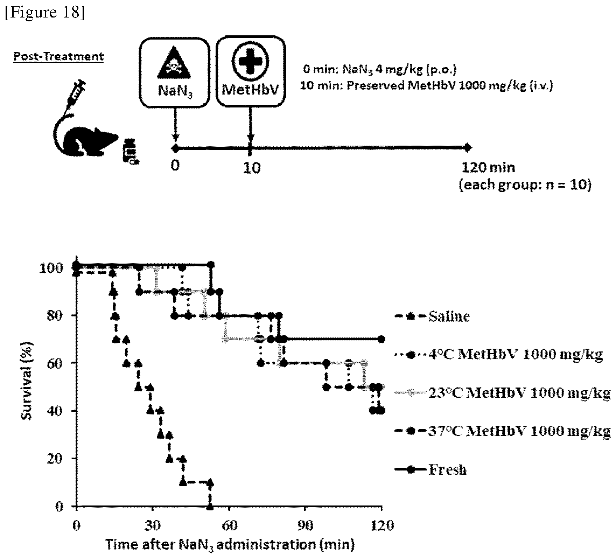

[Figure 18] Figure 18 shows the survival rate of mice, which were post-treated with preserved MetHbV after administration of NaN₃ (40 mg/kg, oral administration) once. The upper view shows an administration schedule of the preserved MetHbV and NaN₃. The lower view shows a change over time in the survival rate of the mice. Ten minutes after administration of the mice with NaN₃, MetHbV (Fresh MetHbV) that had not been preserved after preparation, MetHbV (4°C MetHbV) preserved at 4°C for 1 year, MetHbV (23°C MetHbV) preserved at 23°C to 28°C for 1 year, or MetHbV (37°C MetHbV) preserved at 37°C for 1 year was intravenously administered in an amount of 1000 mg/kg each to the mice.

### Description of Embodiments

Hereafter, the embodiments for carrying out the present invention will be described.

A first embodiment relates to a medicament for cyanide poisoning, hydrogen sulfide poisoning, and azide poisoning, wherein the medicament comprises, as an active ingredient(s), one or more selected from the group consisting of a heme protein capable of binding to cyanide ions (CN⁻), hydrogen sulfide ions (HS⁻) and azide ions (N₃⁻), a substance containing a heme protein capable of binding to CN⁻, HS⁻ and N₃⁻, and a heme derivative capable of binding to CN⁻, HS⁻ and N₃⁻.

In this context, the heme protein is formed by binding between a porphyrin derivative referred to as "heme" and a protein. The heme protein includes hemoglobin, myoglobin, cytochrome, and the like. Hemoglobin, a representative heme protein, has a tetrameric structure composed of 2 types of two subunits (i.e. two α subunit and two β subunit). Each subunit of the hemoglobin consists of a polypeptide referred to as "globin" and heme, and in general, divalent iron ions (Fe²⁺) are coordinated to the heme. When the iron coordinated to the heme is oxidized-form trivalent iron ions (Fe³⁺), it is called "methemoglobin (MetHb)." Differing from hemoglobin, methemoglobin cannot bind to oxygen, and thus, the methemoglobin does not have oxygen carrying capacity in a living body. The present inventors have found that methemoglobin has binding ability to CN⁻, HS⁻ and N₃⁻, specifically, the methemoglobin is a "heme protein capable of binding to CN⁻, HS⁻ and N₃⁻." The present inventors have studied whether or not MetHb or a MetHb-containing substance can be utilized for the prophylaxis or treatment of cyanide poisoning, hydrogen sulfide poisoning, and azide poisoning. As a result, the present inventors have revealed that such MetHb or a MetHb-containing substance can be actually utilized therefor.

In the present embodiment, the "heme protein capable of binding to CN⁻," the "heme protein capable of binding to HS⁻" and the "heme protein capable of binding to N₃⁻" include polymerized proteins thereof and high-molecular-weight proteins thereof. When the heme protein capable of binding to CN⁻, HS⁻ and N₃⁻ is MetHb, the MetHb includes intramolecularly cross-linked MetHb that prevents MetHb from dissociation into individual subunits, intermolecularly cross-linked MetHb formed by intermolecularly cross-linking multiple MetHb with glutaraldehyde or active raffinose in order to increase the molecular weight of MetHb, polymer-bound MetHb formed by chemically binding polyethylene glycol, dextran or albumin to MetHb, in addition to simple MetHb.

Hb is not limited to Hb purified from human blood, and the Hb used herein may include Hb purified from livestock animals (e.g. a swine, a bovine, etc.) and other organisms (e.g. lugworm, earthworm, etc.), and genetically modified Hb. In addition, a heme protein containing heme to which divalent iron ions (Fe²⁺) are coordinated, such as myoglobin (Mb) or cytochrome, can also be used as a raw material.

The heme derivative is a substance formed by allowing various functional groups to covalently bind to heme, wherein the substance is capable of binding to a gas molecule in the case of divalent iron ions (Fe²⁺) or is capable of binding to CN⁻ in the case of trivalent iron ions (Fe³⁺), under a hydrophobic environment or as a result of chemical modification that is addition of a basic ligand. For example, an aqueous solution of the heme derivative, a heme derivative-carrying albumin or liposome, a micelle thereof, and the like can also be utilized.

Moreover, the "substance containing a heme protein capable of binding to CN⁻, HS⁻ and N₃⁻" is a substance that contains a heme protein capable of binding to CN⁻, HS⁻ and N₃⁻. When the heme protein capable of binding to CN⁻, HS⁻ and N₃⁻ is MetHb, examples of a MetHb-containing substance may include a methemoglobin-containing erythrocyte (met RBC(Red Blood Cell)) that contains hemoglobin converted to methemoglobin, and a MetHb-containing capsule, in which MetHb is contained in a capsule. Examples of materials for this capsule may include polymer thin films prepared from materials comprising: macromolecules such as polystyrene, gum Arabic, nylon, and silicone; organism-derived materials such as gelatin; polymers of poly ε caprolactam or polyethylene glycol with biodegradable polymers such as polylactic acid or polyglycolic acid; polysaccharides; and copolymers of amino acid polymers. Further, other examples of materials for the capsule may include, but are not limited to, hydrogel, silica gel, a polyionic complex, a polymersome, a noisome, and a liposome. As such a MetHb-containing capsule, a methemoglobin vesicle (MetHbV), in which MetHb is encapsulated in a liposome, is preferable. As one example of the MetHb-containing capsule in which MetHb is encapsulated in a liposome, the structure of MetHbV consists of 4 components, namely, 1,2-dipalmitoyl-sn-glycero-3-phosphatidylcholine (DPPC), cholesterol, 1,5-O-dihexadecyl-N-succinyl-L-glutamate (DHSG), and 1,2-distearoyl-sn-glycero-3-phosphatidylethanolamine-N-PEG5000 (DSPE-PEG5000).

MetHb and a MetHb-containing substance can be prepared by oxidizing Hb or a Hb-containing substance. Hereafter, a method of preparing MetHbV that is one example of the MetHb-containing substance will be mainly described. MetHb can be prepared by the same method.

MetHbV can be prepared, for example, by allowing a hemoglobin vesicle produced according to a known method (e.g. Non Patent Literature 6 and Non Patent Literature 7, etc.) to react with an oxidizer. The oxidizer is not particularly limited, and for example, sodium nitrite, 4-dimethylaminophenol, hydrogen peroxide, K₃[Fe(CN)₆], etc. can be used.

Specifically, a solvent (or a dispersant; the same applies hereafter) is placed in a vessel (a glass, a plastic, etc.), and an oxidizer is then added thereto, so that the oxidizer is dissolved or dispersed in the solvent. The solvent is generally a normal saline, but is not limited thereto. Subsequently, a hemoglobin vesicle (HbV) is added to the vessel in which the oxidizer and the solvent are placed. When 1 M NaNO₂ is used herein as an oxidizer, the mixing ratio between the HbV and the oxidizer is 10 : 1 to 100 : 1, preferably 20 : 1 to 80 : 1, and more preferably 40 : 1 to 60 : 1, at a volume ratio of the HbV : the oxidizer (HbV : 1 M NaNO₂). The amount of the solvent is not particularly limited, as long as the HbV and the oxidizer are sufficiently mixed therein. The reaction time after the mixing of the HbV, the oxidizer and the solvent can be adjusted depending on the concentration of the oxidizer used. As the concentration of the oxidizer increases, the reaction can be completed in a short time. Thereby, the HbV reacts with the oxidizer, so that 99% or more of the HbV can be converted to MetHbV.

Thereafter, according to ultracentrifugation, or according to centrifugation after addition of high-molecular-weight dextran, gel filtration (GPC) or ultrafiltration, unreacted oxidizer is removed, and MetHbV is purified.

In the case of performing ultracentrifugation, the ultracentrifugation operation may be carried out one or more times or may also be carried out multiple times (for example, 2 or 3 times), although it depends on the type of an ultracentrifugation machine used. For instance, in a case where MetHbV is prepared at a mixing ratio (volume ratio) of HbV : 1 M NaNO₂ = 50 : 1, the ultracentrifugation conditions are preferably 50,000 x g and approximately 20 minutes.

In the case of performing centrifugation after addition of high-molecular-weight dextran, for example, high-molecular-weight dextran (e.g. Dextran from Leucostoc spp., Mr. 450,000 to 650,000) is dissolved in a phosphate-buffered saline (PBS) to produce a high-molecular-weight dextran solution. At this time, the concentration of dextran in the high-molecular-weight dextran solution is 10% by weight to 30% by weight, and preferably about 20% by weight. Thereafter, to the oxidized HbV-dispersed solution, PBS in an amount equal to the HbV-dispersed solution, and an aqueous solution of dextran that is in an amount 0.6 equal to the HbV-dispersed solution, were added. The mixed solution is treated at approximately 3000 g x 20 min, using a centrifuge. As a result of the excluded volume effect of the high-molecular-weight dextran, the HbV is precipitated. After the removal of a supernatant, PBS is added to the residue to re-disperse the precipitate, and an excessive oxidizer is removed.

In a case where ultrafiltration is carried out after addition of an oxidizer to HbV, an ultrafiltration film (for example, Biomax manufactured by Merck Millipore; cut off Mw. 1000 kDa; filtration area: 0.1 m²) is used, and the oxidized HbV is circulated at room temperature. While maintaining the circulating volume, PBS was gradually added thereto in an amount that is about 6 times higher than the initial volume of HbV, and a filtrate containing a free oxidizer is discharged. The pressure on the outlet side of the circulating fluid is maintained, such that it does not exceed, for example, 0.1 MPa.

In a case where gel filtration is carried out after addition of an oxidizer to HbV, for example, a gel filtration column filled with Sepharose CL-4B is washed with PBS, and HbV allowed to react with an oxidizer is then developed. First, MetHbV having a large particle size is flown out, and an unreacted oxidizer is then flown out. Thus, the unreacted oxidizer can be removed.

Alternatively, MetHbV can also be prepared by allowing HbV to bind to NO, and then allowing the HbV to come into contact with O₂. Specifically, for example, a HbV-dispersed solution is deaerated with N₂, and is then allowed to come into contact with NO, so as to generate HbNO. Again, excess NO is deaerated with N₂, and is then allowed to come into contact with O₂, thereby preparing MetHbV.

An aqueous solvent whose osmotic pressure, etc. or component concentration are physiologically accepted is added to the prepared MetHbV-dispersed solution, so that the MetHbV-dispersed solution can be adjusted to have a desired concentration and a desired viscosity.

Otherwise, MetHbV can also be prepared by encapsulating MetHb formed by oxidation of hemoglobin into a vesicle. In order to encapsulate MetHb into a vesicle, encapsulation can be carried out according to the aforementioned methods (Non Patent Literature 6 and Non Patent Literature 7, etc.).

In MetHbV, the zeta potential takes a negative value. The zeta potential of MetHbV is preferably -16.00 to -10.00, and more preferably -15.00 to -11.00. In addition, the particle size of MetHbV is preferably 100 to 300 nm, more preferably 200 to 300 nm, and further preferably 240 to 250 nm. The zeta potential and the particle size can be measured by dynamic light scattering. The maximum absorption wavelength of the Soret band absorption spectrum of preferred MetHbV is 400 to 410 nm, and is generally around 408 nm. The absorption spectrum of MetHbV can be measured by ultraviolet-visible spectroscopy.

CN⁻, HS⁻ and N₃⁻ pass through a vesicle (a lipid bimolecular membrane) of MetHbV, so that they can bind to the MetHb in the vesicle, and the prophylactic or therapeutic effects of the MetHb on each poisoning can be exhibited.

When the medicament according to the present embodiment is administered to a subject (a person who is at risk of developing cyanide poisoning, hydrogen sulfide poisoning, or azide poisoning) before the onset of each poisoning, the present medicament can suppress the onset of the symptoms of each poisoning. On the other hand, when the medicament according to the present embodiment is administered to a patient (a person who has developed cyanide poisoning, hydrogen sulfide poisoning, or azide poisoning) after the onset of the poisoning, the present medicament can improve the symptoms of each poisoning and can restore a normal condition in the patient. Therefore, the medicament according to the present embodiment can be used as a therapeutic drug and a prophylactic drug for cyanide poisoning, hydrogen sulfide poisoning, and azide poisoning.

As such a medicament according to the present embodiment, the active ingredient itself (i.e. a heme protein capable of binding to CN⁻, HS⁻ and N₃⁻, etc.) may be administered. However, the present medicament may also be administered in the form of a pharmaceutical composition comprising one or two or more common pharmaceutical additives, as well as the active ingredient. The medicament and the pharmaceutical composition according to the present embodiment (hereinafter also referred to as a "medicament, etc.") may also comprise existing active ingredients whose effects of improving the symptoms of cyanide poisoning, hydrogen sulfide poisoning and azide poisoning have been confirmed, such as, for example, Na₂S₂O₃ and hydroxocobalamin, as well as the aforementioned active ingredient. The mixed amount of such existing active ingredients is, as an example, 1% by weight or more, and 90% by weight or less, or 50% by weight or less, with respect to the weight of the active ingredient.

The dosage form of the medicament, etc. according to the present embodiment is not particularly limited, and examples of the dosage form may include a drip infusion and an injection. Liquid formulations such as a drip infusion and an injection can be prepared by dispersing an active ingredient, for example, MetHb or a MetHb-containing substance, in a normal saline, and then adding a buffer agent or a preservative to the obtained solution, as necessary. The preferred viscosity range of such a drip infusion or an injection is 1.0 to 5.0 cP. When the active ingredient is dispersed in a normal saline to adjust the resulting drip fusion or injection to the above-described viscosity range, the concentration of the active ingredient that is, for example, MetHbV, is preferably 15 g/dL or less, and is more preferably 10 g/dL or less. When the concentration of MetHbV to be dispersed in a normal saline is within the aforementioned range, the viscosity of the MetHbV-dispersed solution is moderately regulated, and thus, the MetHbV-dispersed solution is easily administered.

Moreover, when the medicament according to the present embodiment comprises MetHbV, the MetHbV may not only be MetHbV that has been prepared at the time of use, but may also be MetHbV that has been preserved after the preparation thereof. The preservation period of MetHbV may be a long period of time, such as, for example, about 1 year. The temperature applied upon preservation of MetHbV may be, for example, 0°C or higher and 40°C or lower, and preferably 4°C or higher and 37°C or lower.

The types of pharmaceutical additives used in the production of the medicament, etc. according to the present embodiment, the ratio of the pharmaceutical additives to the active ingredient, and the method for producing the medicament, etc. can be selected, as appropriate, by those skilled in the art, depending on the form of the medicament, etc. As such pharmaceutical additives, inorganic or organic substances, or solid or liquid substances can be used. Such pharmaceutical additives can be mixed in an amount of 1% by weight or more and 90% by weight or less, with respect to the weight of the active ingredient.

The applied dose and the number of doses of the medicament, etc. according to the present embodiment are not particularly limited, and can be selected, as appropriate, at a physician's discretion, depending on conditions such as the degree of progression of the symptoms of cyanide poisoning, hydrogen sulfide poisoning or azide poisoning in a therapeutic or prophylactic subject, and the body weight, age, and the like of an administration subject.

When the present medicament is used as an injection or the like, for example, 0.001 to 1000 mg of the medicament (relative to the weight of a hemoglobin vesicle) may be continuously or intermittently administered to an adult at a daily dose. With regard to the timing of administration, the medicament may be administered as quickly as possible after the onset of cyanide poisoning, hydrogen sulfide poisoning or azide poisoning has been confirmed, or may also be administered prophylactically, before the subject is put in a situation in which cyanide poisoning, hydrogen sulfide poisoning or azide poisoning is likely to be developed.

The medicament, etc. according to the present embodiment may be provided in the form of a kit, together with an instruction manual regarding an administration method and the like. By this kit, the drug is supplied by being enclosed in a vessel, in which the activity of the structural components of the medicament, etc. is effectively sustained for a long period of time, the medicament is not adsorbed on the inner side of the vessel, and the vessel is produced with a material that does not alter the structural components. For instance, the medicament, etc. may be supplied by being enclosed in a glass vessel or a plastic vessel.

Further, instructions for use may be included with the kit. The instructions for use of the kit may be printed out on a paper or the like, or may also be preserved in an electromagnetically readable medium such as CD-ROM or DVD-ROM, and the instructions for use may be then provided to a user.

A second embodiment relates to a method for treating or preventing cyanide poisoning, hydrogen sulfide poisoning, and azide poisoning, wherein the method comprises administering a medicament or a pharmaceutical composition for cyanide poisoning, comprising, as an active ingredient(s), one or more selected from the group consisting of a heme protein capable of binding to CN⁻, HS⁻ and N₃⁻, a substance containing a heme protein capable of binding to CN⁻, HS⁻ and N₃⁻, and a heme derivative capable of binding to CN⁻, HS⁻ and N₃⁻, to a patient with cyanide poisoning or a subject who is likely to develop cyanide poisoning.

In this context, the term "treat" means to inhibit or alleviate progression and deterioration of the pathological conditions of a patient who has already developed cyanide poisoning, hydrogen sulfide poisoning or azide poisoning, and it is a treatment for the purpose of inhibiting or alleviating progression and deterioration of the developed cyanide poisoning.

On the other hand, the term "prevent" means to previously inhibit the onset of cyanide poisoning, hydrogen sulfide poisoning or azide poisoning in a patient who is at risk of developing the aforementioned poisoning, and it is a treatment for the purpose of previously inhibiting the onset of cyanide poisoning.

The therapeutic and prophylactic targets are "mammals." The "mammals" are used herein to mean any given animals classified into Mammalia, and thus, are not particularly limited. Examples of such mammals may include companion animals such as a dog and a cat, and livestock animals such as a bovine, a swine, sheep and a horse. A particularly preferred "mammal" is a human.

A third embodiment relates to use of a heme protein capable of binding to CN⁻, HS⁻ and N₃⁻, a substance containing a heme protein capable of binding to CN⁻, HS⁻ and N₃⁻, and a heme derivative capable of binding to CN⁻, HS⁻ and N₃⁻, for the production of a medicament or a pharmaceutical composition for cyanide poisoning, hydrogen sulfide poisoning, and azide poisoning. Regarding a detailed explanation of the present embodiment, please refer to the explanation for the first embodiment.

When the present description is translated into English and the translation document includes singular terms with the articles "a," "an," and "the," these terms include not only single items but also multiple items, unless otherwise clearly specified from the context.

### Examples

Hereinafter, the present invention will be described in the following examples. However, these examples are not intended to limit the scope of the present invention. In the present examples, the symbol"%" indicates "% by weight," unless otherwise specified.

### 1. Materials and Experimental Methods

### 1-1. Experimental Samples

HbV and an empty vesicle were produced based on previous reports (see Non Patent Literature 6 and Non Patent Literature 7). As other reagents and solvents, commercially available special grade products were used, and ultrapure water was used as water that was to be used as a solvent.

### 1-2. Production of MetHbV

MetHbV was produced by oxidizing HbV. Sodium nitrite (NaNO₂) was dissolved in a normal saline to a concentration of 1 M, and 100 µL of the thus prepared solution was added to 5 mL of a HbV solution (10 g Hb/dL), and was then reacted for 24 hours so as to produce MetHbV (Non Patent Literature 8). After completion of the reaction, the ratio of metHb was measured (as described later), and only MetHbV, in which the ratio of metHb was 95% or more, was used as a sample. The produced MetHbV solution was repeatedly washed with a normal saline three times according to ultracentrifugation (50,000 g, 30 min) (Non Patent Literature 9), so that excess NaNO₂ was removed. The MetHbV was dispersed in a normal saline to adjust it to a final concentration of interest.

### 1-3. Observation with Transmission Electron Microscope (TEM)

MetHbV (45 µM or 0.3 g Hb/dL) was dropped onto a TEM grid, and was then subjected to negative staining with 2% samarium acetate. Thereafter, an excess liquid was removed, and the resultant was then observed using JIM-1230 manufactured by JEOL, at an acceleration voltage of 80 kV.

### 1-4. Measurement of ζ Potential

The ζ potential of MetHbV was measured by diluting MetHbV with a normal saline to 0.1 g Hb/dL, and then applying dynamic light scattering (DLS) (ELSZ-2000Z, manufactured by Otsuka Electronics Co., Ltd.).

### 1-5. Measurement of Particle Size

The particle size and the poly dispersity index (PDI) of MetHbV were measured by diluting a MetHbV sample with a normal saline to 0.1 g Hb/dL, and then using Zetasizer Nano Z manufactured by Malvern Panalytical.

### 1-6. Measurement of Absorption Spectrum Shifts

MetHbV (5.2 µM) was mixed with NaCN (52 µM), and changes in the spectra were then measured using Infinite (registered trademark) M1000 PRO (manufactured by TECAN).

### 1-7. Production of MetHbV for Cell Experiments

MetHbV was produced according to the same production procedures as those for the MetHbV used in evaluation of the physical characteristics. However, RPMI-1640(-) was used, instead of a normal saline, in dilution and washing.

### 1-8. Production of Empty Vesicle

For the purpose of the replacement of a normal saline contained in a vesicle (EV), an empty vesicle (EV) was diluted with an excessive amount of RPMI-1640(-), and the resultant was stirred for 24 hours and was then left. The replaced empty vesicle solution was subjected to ultracentrifugation (100,000 g, 1 h) (Non Patent Literature 10), and was then dispersed in RPMI-1640(-), so as to adjust it to a final concentration of interest.

### 1-9. Production of metHb

MetHb was produced by oxidizing OxyHb. Sodium nitrite (NaNO₂) was dissolved in RPMI-1640 to a concentration of 1 M, and 100 µL of the thus prepared solution was added to 5 mL of a Hb solution (10 g/dL), and was then reacted for 24 hours so as to produce metHb. After completion of the reaction, the ratio of metHb was measured (as described later), and only the reaction mixture, in which the ratio of metHb was 95% or more, was used as a sample. The produced metHb solution was subjected to filter filtration (10 kDa), so that excess NaNO₂ was removed. The metHb was dissolved in RPMI-1640 to adjust it to a final concentration of interest.

### 1-10. Production of OxyHbV

OxyHbV was produced by bubbling O₂ gas through a HbV solution for 5 minutes. OxyHbV was subjected to ultracentrifugation and was then dissolved in RPMI-1640, so as to adjust it to a final concentration of interest.

### 1-11. Production of Methemoglobin-Containing Erythrocyte (Met RBC)

Blood was collected from ddY mice, and was then subjected to centrifugation (3000 rpm, 20 min), so that plasma and leukocyte membrane were removed. The obtained erythrocytes were re-suspended in an equal amount of normal saline, and 100 µL of an NaNO₂ solution adjusted to 1 M was then added to 5 mL of the erythrocyte suspension. The obtained mixture was gently stirred for 10 minutes to produce met RBCs. The ratio of metHb in the erythrocytes was measured using a blood gas analyzer (Rapid point 500, manufactured by Siemens), the erythrocytes, in which the ratio of metHb was 95% or more, were used as a sample. Thereafter, washing with a normal saline and centrifugation (3000 rpm, 20 min) were repeated three times, so as to produce washed met RBCs. The Hb concentration was measured using FUJI DRI-CHEM (FUJIFILM Wako Pure Chemical Corporation), and was then adjusted to a concentration of interest by addition of a normal saline.

### 1-12. Cell Culture

PC-12 cells as rat adrenal pheochromocytoma were purchased from JCRB Cell Bank, National Institutes of Biomedical Innovation, Health and Nutrition, and were then seeded on a collagen-coated cell dish (Iwaki, φ = 100 mm), using RPMI-1640 supplemented with deactivated 5% (v/v) FBS, 10% horse serum, and antibiotics (100 units/mL penicillin and 100 µg/mL streptomycin) (hereinafter referred to as "RPIMI-1640(+)"). Thereafter, the cells were subjected to a static culture at 37°C in a 5% CO₂ concentration. The semi-confluent cells were treated with trypsin and centrifugation (1000 rpm, 5 minutes), and were then re-suspended in RPMI-1640(+), so that the cells were sub-cultured.

### 1-13. Cytotoxicity Test

The PC-12 cells were seeded on a collagen-coated 96-well culture plate (Sumitomo Bakelite Co., Ltd.) to result in a density of 2 × 10⁴ cells/well, and were then culture for 24 hours for adhesion. Thereafter, the medium was removed, and 50 µL each of an NaCN solution prepared by dissolving NaCN in RPMI-1640(-) and then adjusting obtained solution to each concentration (i.e. 0, 2.5, 5, 10, 15, 20, 30, 40, 80 and 160 mM) was then added to each well. Immediately after addition of the NaCN solution, 50 µL of each antidote adjusted to each concentration was added to each well, so that the volume of the solution in each well was set to be 100 µL and the final NaCN concentrations were set to be 0, 1.25, 2.5, 5, 7.5, 10, 15, 20, 40 and 80 mM. The thus obtained mixtures were each cultured at 37°C under 100% moist air for 1 hour.

### 1-14. Establishment of Antidote Treatment Groups

For the purpose of confirming dose-dependent effects, 6 types of additive amounts were set to all groups, namely, the amounts of Hb were set to be 0, 2.5, 5, 10, 15, and 20 g/dL (0, 0.39, 0.78, 1.6, 2.3, and 3.1 mM), or other additive amounts corresponding thereto, were determined. The following groups were established: a MetHbV group used for the purpose of evaluation of effectiveness; two groups for which a nitrous acid-based drug (sodium nitrite) whose effectiveness had already been confirmed was used, namely, a metHb group acting as a result of conversion of Hb to metHb, and a met RBC group; and an OHCbl group having another action mechanism. Moreover, two negative control groups, namely, an OxyHbV group and a sodium thiosulfate group were established. Furthermore, an empty vesicle group consisting only of a lipid bilayer, in which Hb was not enclosed, was established. The groups were each established in the amount of a lipid equivalent to the embedding of Hb, and a total of 7 groups were prepared.

### 1-15. Cell Viability Test

The cells were cultured for 1 year, and a mixed solution of NaCN and an antidote was then removed. The inside of each well was washed with RPMI-1640(-) three times. After addition of 100 µL of RPMI-1640, 10 µL of CCK-8 (Cell Counting Kit-8, Dojindo Laboratories) was added to the resultant, and the obtained mixture was then cultured for 2 hours. Thereafter, the absorbance at 450 nm was measured using a microplate reader (Infinite M1000, Tecan).

### 1-16. Calculation of EC₅₀

Using the results obtained by the cell viability test, EC (Effective Concentration) in individual groups was calculated with reference to the report of Gu et al. (Non Patent Literature 10).

### 1-17. Experimental Animals

### 1-17-1. Cyanide Poisoning Mouse Models

Eight-week-old ddY-strain female mice (25 to 27 g) were purchased from Japan SLC, Inc., and after preliminary breeding for 1 week, the mice were used in experiments.

### 1-17-2. Hydrogen Sulfide Poisoning Mouse Models and Azide Poisoning Mouse Models

Eight-week-old ddY-strain female mice (25 to 27 g) were purchased from Japan SLC, Inc., and after preliminary breeding for 1 week, the mice were used in experiments.

### 1-18. Studies Using Cyanide Poisoning Mouse Models

### 1-18-1. Production of Cyanide Poisoning Mouse Models

The ddY mice were subjected to fasting for 4 hours. A NaCN solution dissolved in a normal saline was adjusted to an administration volume of 10 mL/kg, and was then orally administered to the mice, using a sonde (Natsume Seisakusho Co., Ltd., 23G) under no anesthesia.

### 1-18-2. Establishment of Antidote Pre-Administration Treatment Groups

For the purpose of confirming dose-dependent effects, 4 MetHbV groups were established, namely, 500, 1000, 1500, 2000 mg Hb/kg MetHbV administration groups. Moreover, an empty vesicle group consisting only of a lipid bilayer, in which Hb was not enclosed, was established. The amount of lipid in the empty vesicle was determined to be equal to the amount of a vesicle in the case of administration of 1000 mg/kg MetHbV. As a control group, a group, to which an equal amount of normal saline was administered, was established. Furthermore, as control groups, to which existing drugs whose effectiveness had been confirmed were administered, 4 groups, namely, an NaNO₂ (17 mg/kg) group, an NaNO₂ (17 mg/kg) + Na₂S₂O₃·5H₂O (61 mg/kg) group, an Na₂S₂O₃·5H₂O (61 mg/kg) group, and an OHCbl (330 mg/kg) group were established. Thus, a total of 10 groups were prepared. The applied dose of the existing drug was determined, so that it became a molar ratio that was equal to the orally administered NaCN. That is to say, the LD₉₀ value of NaCN to the mice was determined to be 12 mg/kg, and the applied dose of all of the existing drugs was set to be 0.245 mmol/kg.

### 1-18-3. Production of Antidote Pre-Treated Cyanide Poisoning Mouse Models

The applied volume of each antidote was determined to be 10 mL/kg, and each antidote was administered to the mice via caudal vein under 3% isoflurane inhalation anesthesia. After completion of the administration of each antidote, the anesthesia was quickly terminated, and the mice were awakened. Five minutes later, an NaCN solution was orally administered to the mice at a dose of LD₅₀ (8.5 mg/kg) or LD₉₀ (12 mg/kg). The mice that were not returned to a complete awaking state within 5 minutes due to the influence of the anesthesia were not used in the present study. Regarding the awakening state, the recovery of a reflex reaction was confirmed according to the following definitions of "Awake."

### 1-18-4. Establishment of Antidote Post-Administration Treatment Groups

Two MetHbV groups, namely, a 1000 mg/kg administration group, and a 1000 x 2 mg/kg (total: 2000 mg Hb/kg) administration group, in which MetHbV was administered at a dose of 20 mL/kg that was two times the aforementioned dose, were established. In addition, a MetHbV1000 mg/kg + Na₂S₂O₃·5H₂O (61 mg/kg) group was established as a combined use with the existing drug. Furthermore, as with the pre-administration treatment groups, 4 groups, namely, an NaNO₂ group, an NaNO₂ + Na₂S₂O₃·5H₂O group, an Na₂S₂O₃·5H₂O group, and an OHCbl group were established as existing drug control groups. Thus, a total of 7 groups were prepared.

### 1-18-5. Production of Antidote Post-Treated Cyanide Poisoning Mouse Models

An NaCN solution was orally administered to the mice at a dose of LD₉₀ (12 mg/kg), and 5 minutes or 10 minutes later, each antidote was administered to the mice that were in a Coma state via caudal vein.

### 1-18-6. Measurement of time from antidote administration until awakening

After each antidote had been administered to the mice that were in a Coma state due to cyanide poisoning, and the time required from administration of each antidote until the mice became awakening (Awake) was then measured. Besides, "Coma" was defined to be the loss of a righting reflex, and the time required from a mouse being placed on a board in a state of lying on back (supine position) until the time point at which the mouse could not returned to a normal state that was a prone state (prone position) by itself was measured (Non Patent Literature 11). "Awake" was defined to be a state in which the righting reflex of the mouse is recovered from the Coma and it functions. In order to avoid a false positive reaction due to reflection such as convulsion, an identical trial was carried out on each mouse twice.

### 1-19. Studies Using Hydrogen Sulfide Poisoning Mouse Models

### 1-19-1. Antidote Pre-Treatment

Each antidote was intravenously administered to 9-week-old ddY female mice under isoflurane anesthesia, 5 minutes before administration of hydrogen sulfide (-5 minutes). Sodium hydrosulfide (NaHS) (35 mg/kg) dissolved in a normal saline was subcutaneously administered to the mice, and the survival rate of the mice was tracked until 60 minutes after administration of NaHS. The experiment was carried out at a number of n = 10 in each group. Besides, 35 mg/kg NaHS exhibits toxicity that was 90% lethal dose (LD₉₀) or higher. Administration groups of individual antidotes and a normal saline (control) were established as follows.
MetHbV administration groups:
   500 mg Hb/kg (5 mL/kg)
   1000 mg Hb/kg (10 mL/kg)
Sodium nitrite (NaNO₂) administration groups:
   17 mg/kg (10 mL/kg)
   34 mg/kg (10 mL/kg)
Hydroxocobalamin (OHCbl) administration group: 330 mg/kg (10 mL/kg)
Saline (normal saline) administration group: 10 mL/kg

### 1-19-2. Antidote Post-Treatment (Therapeutic)

Sodium hydrosulfide (NaHS) (35 mg/kg) dissolved in a normal saline was subcutaneously administered to 9-week-old ddY female mice. Five minutes after administration of NaHS, each antidote was intravenously administered to the mice under isoflurane anesthesia, and the survival rate of the mice was tracked until 60 minutes after administration of NaHS. The experiment was carried out at a number of n = 10 in each group. Administration groups of individual antidotes were established as follows.
MetHbV administration groups:
   1000 mg Hb/kg (10 mL/kg)
   1000 × 2 mg Hb/kg (20 mL/kg)
Sodium nitrite (NaNO₂) administration group: 34 mg/kg (10 mL/kg)
Hydroxocobalamin (OHCbl) administration group: 330 mg/kg (10 mL/kg)

### 1-20. Studies Using Azide Poisoning Mouse Models

### 1-20-1. Antidote Pre-Treatment

Each antidote was intravenously administered to 9-week-old ddY female mice under isoflurane anesthesia, 5 minutes before administration of sodium azide (NaN₃) (-5 minutes). Sodium azide (NaN₃, 40 mg/kg) dissolved in a normal saline was orally administered to the mice, and the survival rate of the mice was tracked until 120 minutes after administration of sodium azide (NaN₃). The experiment was carried out at a number of n = 10 in each group. Besides, 40 mg/kg NaN₃ exhibits toxicity that was about 90% lethal dose (LD₉₀) or higher. Administration groups of individual antidotes and a normal saline (control) were established as follows.
MetHbV administration groups:
   1000 mg Hb/kg (10 mL/kg)
   1000 × 2 mg Hb/kg (20 mL/kg)
Sodium nitrite (NaNO₂) administration group: 17 mg/kg (10 mL/kg)
Hydroxocobalamin (OHCbl) administration group: 330 mg/kg (10 mL/kg)
Saline (normal saline) administration group: 10 mL/kg

### 1-20-2. Antidote Post-Treatment (Therapeutic)

Sodium azide (NaN₃) (40 mg/kg) dissolved in a normal saline was orally administered to 9-week-old ddY female mice. Ten minutes after administration of NaN₃, each antidote was intravenously administered to the mice under isoflurane anesthesia, and the survival rate of the mice was evaluated until 120 minutes after administration of NaN₃. The experiment was carried out at a number of n = 10 in each group. Administration groups of individual antidotes were established as follows.
MetHbV administration groups:
   500 mg Hb/kg (5 mL/kg)
   1000 mg Hb/kg (10 mL/kg)
   1000 × 2 mg Hb/kg (20 mL/kg)
Sodium nitrite (NaNO₂) administration group: 17 mg/kg (10 mL/kg)
Hydroxocobalamin (OHCbl) administration group: 330 mg/kg (10 mL/kg)
Saline (normal saline) administration group: 10 mL/kg

### 1-21. Confirmation of Stability and Antidotal Effect of Preserved MetHbV

### 1-21-1. Stability of Preserved MetHbV

### Preservation Conditions of MetHbV

A MetHbV suspension (10 g Hb/dL) was placed in a glass vial, and was then preserved at 4°C (cold storage), at 23°C to 28°C (room temperature) or at 37°C (warm) for 1 year (12 months).

### Physicochemical Characteristics of MetHbV Preserved for Long Period of Time (1 Year (12 Months))

Using Zetasizer Nano ZS (Malvern Panalytical Ltd., UK) and ELSZ2KOP Zeta Potential Analyzer (Otsuka Electronics Co., Ltd., Japan), the particle size, poly dispersity index (PDI), and ζ potential of MetHbV were measured.

### 1-21-2. Confirmation of Effectiveness against Cyanide Poisoning

Sodium cyanide (NaCN) (12 mg/kg) dissolved in a normal saline was orally administered to 9-week-old ddY female mice. Ten minutes after administration of NaCN, preserved MetHbV was intravenously administered to the mice, and the survival rate of the mice was evaluated until 60 minutes after administration of NaCN. The experiment was carried out at a number of n = 10 in each group. Administration groups were established as follows.
4°C Preserved MetHbV administration group: 1000 mg Hb/kg (10 mL/kg)
23°C to 28°C Preserved MetHbV administration group: 1000 mg Hb/kg (10 mL/kg)
37°C Preserved MetHbV administration group: 1000 mg Hb/kg (10 mL/kg)

### 1-21-3. Confirmation of Effectiveness against Hydrogen Sulfide Poisoning

Sodium hydrosulfide (NaHS) (35 mg/kg) dissolved in a normal saline was subcutaneously administered to 9-week-old ddY female mice. Five minutes after administration of NaHS, preserved MetHbV was intravenously administered to the mice, and the survival rate of the mice was evaluated until 60 minutes after administration of NaHS. The experiment was carried out at a number of n = 10 in each group. Administration groups were established as follows.
4°C Preserved MetHbV administration group: 1000 mg Hb/kg (10 mL/kg)
23°C to 28°C Preserved MetHbV administration group: 1000 mg Hb/kg (10 mL/kg)
37°C Preserved MetHbV administration group: 1000 mg Hb/kg (10 mL/kg)

### 1-21-4. Confirmation of Effectiveness against Azide Poisoning

Sodium azide (NaN₃) (40 mg/kg) dissolved in a normal saline was subcutaneously administered to 9-week-old ddY female mice. Ten minutes after administration of NaN₃, preserved MetHbV was intravenously administered to the mice, and the survival rate of the mice was evaluated until 120 minutes after administration of NaN₃. The experiment was carried out at a number of n = 10 in each group. Administration groups were established as follows.
4°C Preserved MetHbV administration group: 1000 mg Hb/kg (10 mL/kg)
23°C to 28°C Preserved MetHbV administration group: 1000 mg Hb/kg (10 mL/kg)
37°C Preserved MetHbV administration group: 1000 mg Hb/kg (10 mL/kg)

### 1-22. Ethical Considerations

All animal experiments were carried out with the approval of the Keio University Institutional Animal Care and Use Committee (Approval Nos. 18013-(0), 20046-(0), and 20067-(0)).

### 2. Results

### 2-1. Production of MetHbV and Evaluation of Physicochemical Characteristics

First of all, MetHbV was produced and was then observed by TEM. In addition, the physicochemical characteristics of the particles were evaluated by measuring particle size distribution (particle size, PDI) and ζ potential according to DLS.

First, hemoglobin enclosed in HbV was converted to methemoglobin with sodium nitrite used as an oxidizer, so as to produce MetHbV. The MetHbV particle seen in a TEM image was a monolayer liposome and had a spherical form (Figure 1).

Thereafter, the physicochemical characteristics of the obtained MetHbV were evaluated. As a result, the MetHbV was composed of particles, which had a mean particle size of 240.7 nm and a ζ potential of -13.2 mV and were present in a monodispersed state (Figure 2 and Table 1).

**[Table 1]**

| Physicochemical characteristics of MetHbV. (Particle size, PDI and ζ- potential) | | | |
|---|---|---|---|
| Lot. No. | Particle size (nm) | PDI | ζ- potential (mV) |
| 1 | 245.6±0.9 | 0.092±0.01 | -11.8±0.67 |
| 2 | 234.9±1.1 | 0.093±0.01 | -14.2±0.64 |
| 3 | 241.5±2.7 | 0.090±0.01 | -13.5±0.31 |
| Average | 240.7±4.4 | 0.092±0.00 | -13.2±1.0 |

| | | | |
|---|---|---|---|
| * Each lot has been produced, separately. Values are each indicated with a mean ±S.D (n = 3). | | | |

### 2-2. Evaluation of Formation of MetHbV and Binding Ability to Cyanide by Spectral Change

A spectral change of MetHbV was evaluated according to ultraviolet-visible spectroscopy. HbV was preserved under N₂ substitution and was present in the form of DeoxyHbV. Once HbV came contacted with air, however, it immediately bound to oxygen in the air and exhibited a peak derived from OxyHbV (415 nm). Moreover, by adding sodium nitrite to a HbV solution, the peak wavelength was changed to 408 nm, and formation of MetHbV was confirmed. Furthermore, a sodium cyanide solution was mixed with MetHbV, and formation of cyanide binding type MetHbV (CN-MetHbV) was then evaluated. As a result, the MetHbV-derived peak (408 nm) was shifted to a cyanide binding type MetHbV-derived peak (420 nm), and the binding ability of MetHbV to cyanide was demonstrated (Figure 3).

### 2-3. Cell Evaluation (In Vitro)

### 2-3-1. Evaluation of Cytotoxicity of MetHbV

In order to evaluate the cytotoxicity of MetHbV, a MetHbV solution was added in a different concentration to PC12 cells, and a comparison was then made in terms of cell viability. As a result, a survival rate of approximately 95% was confirmed in a 20 g/dL MetHbV addition group, and thus, MetHbV exhibited almost no toxicity on the cells (Figure 4).

### 2-3-2. Cell-protecting effect of MetHbV against cyanide poisoning

In order to study that MetHbV coordinates with CN⁻ and competitively inhibits the binding of cyanide ions to CcOX in each cell so as to exhibit effectiveness, sodium cyanide (NaCN) was added to the cells, and each antidote was then added thereto. Thereafter, a comparison was made in terms of cell viability. As a result, a control group, to which only NaCN had been added, had a cell viability of approximately 49% upon addition of 10 mM NaCN. In contrast, a 2.5 g/dL MetHbV addition group had a cell viability of approximately 87%, and thus, the survival rate was significantly increased. In addition, the concentration dependency of MetHbV against NaCN was examined. As a result, when 20 mM NaCN was added, a 2.5 g/dL MetHbV addition group had a cell viability of 39%, a 5 g/dL MetHbV addition group had a cell viability of 47%, and a 10 g/dL MetHbV addition group had a cell viability of 77%. Thus, it was confirmed that the cell viability was increased in a MetHbV concentration-dependent manner. On the other hand, as comparisons with the MetHbV addition groups, an OxyHbV addition group, in which hemoglobin in a liposome was not converted to methemoglobin, and an empty vesicle (EV) group, in which hemoglobin was not enclosed in a liposome, were established. As a result, these groups exhibited the same behavior as that of the control group, and thus, it was confirmed that these groups are not associated with detoxification such as coordination of CN⁻. From these results, it was demonstrated that MetHbV exhibits cell protection (antidotal ability), and that, as an action mechanism thereof, the cell protection by MetHbV is caused by coordination of cyanide to methemoglobin in MetHbV (Figure 5).

### 2-3-3. Comparison of cell-protecting effect of MetHbV against cyanide poisoning with those of existing drugs

In order to study the effectiveness of MetHbV against cyanide, the same experiment as that described above was carried out using existing drugs that have been currently used in clinical sites, and a comparison was then made in terms of cell viability. As a result, met RBCs and metHb, which had been prepared with sodium nitrite, exhibited a significantly high survival rate, compared with the control group to which only NaCN had been added. Moreover, hydroxocobalamin (OHCbl), which had been set at a molar ratio equal to that of hemoglobin and performed detoxification by a different action mechanism, exhibited a high survival rate, compared with the control group. In contrast, a sodium thiosulfate (Na₂S₂O₃) addition group exhibited the same behavior as that of the control group, and had no effects. The met RBCs, metHb, and OHCbl addition groups, which were found to have effects, exhibited the same behavior as that of the MetHbV addition groups, in all concentrations. From these results, in the evaluation using the cells, MetHbV was demonstrated to have effectiveness equivalent to that of the existing drugs (Figure 6).

### 2-3-4. Comparison regarding Effective Concentration (EC) of Cyanide under Existence of Each Antidote

In order to make a comparison in terms of the toxicity of sodium cyanide to PC12 cells under the existence of each antidote, studies were conducted using 50% effective concentration (EC₅₀) as an indicator of cytotoxicity. As a result, the EC₅₀ value became 10.53 mM, when the cells were exposed to only NaCN without using an antidote. On the other hand, the EC₅₀ value of a 2.5 g/dL MetHbV addition group became 17.9 mM, and thus, it was confirmed that MetHbV exhibited sufficient effects although it was used in a low concentration such as 2.5 g/dL. Moreover, in the case of met RBCs and metHb exhibiting the same action mechanism as that of MetHbV upon detoxification, the EC₅₀ values of 2.5 g/dL met RBC and metHb addition groups became 20.6 mM and 16.9 mM, respectively, and thus, met RBCs and metHb exhibited the same effects. The OxyHbV addition group and the empty vesicle group, which did not exhibit antidotal ability, merely had EC₅₀ values of 10.3 mM and 10.7 mM, respectively, although they were each added in a high concentration (20 g/dL) (Table 2).

**[Table 2]**

| EC₅₀ of cyanide under existence of each antidote | | | | | | | | |
|---|---|---|---|---|---|---|---|---|
| Added antidotes | | | | | | | | |
| | | MetHbV | met RBC | metHb | OHCbl | OxyHbV | Empty vesicle | Na₂S₂O₃ |
| Conc. (g/dL) | 2.5 | 17.88 (16.62-19.14) | 20.61 (10.64-40.41) | 16.94 (14.33-20.44) | 13.25 (10.15-17.90) | 9.37 (6.14-15.66) | 10.55 (4.36-28.80) | 10.36 (8.65-12.22) |
| | 5 | 19.37 (17.57-21.12) | 22.17 (13.11-40.40) | 19.15 (17.64-20.97) | 17.57 (16.07-19.25) | 9.61 (8.69-10.76) | 10.77 (8.31-14.08) | 10.67 (9.58-11.76) |
| | 10 | 26.02 (25.12-27.01) | 27.91 (21.83-38.06) | 23.35 (17.31-33.72) | 22.54 (20.27-26.09) | 9.52 (8.18-11.40) | 9.52 (7.75-13.01) | 9.98 (7.45-14.82) |
| | 15 | 40.35 (36.71-44.30) | 33.60 (23.95-50.35) | 28.77 (26.68-31.26) | 31.26 (27.63-36.34) | 9.59 (6.87-15.43) | 10.52 (3.63-12.74) | 12.52 (11.33-13.89) |
| | 20 | 43.15 (39.32-48.43) | 40.79 (25.37-43.86) | 31.15 (24.60-45.99) | 34.41 (31.04-38.96) | 10.26 (7.38-17.20) | 10.69 (9.33-12.22) | 12.12 (10.15-15.03) |

| | | | | | | | | |
|---|---|---|---|---|---|---|---|---|
| All EC₅₀ values are represented by mM unit. Inside ( ) represents confidential intervals. | | | | | | | | |

### 2-4. Evaluation Using Animals (In Vivo)

### 2-4-1. Studies Using Cyanide Poisoning Mouse Models

### 2-4-1-1. Evaluation of Effectiveness of MetHbV in Prophylactic Administration (Pre-Treatment)

In order to verify that MetHbV is effective for cyanide poisoning even in an *in vivo* environment, evaluation was carried out using ddY-strain female mice. After administration of MetHbV, NaCN was orally administered to the mice in an amount of 8.5 mg/kg that was a median lethal dose (LD₅₀). As a result, in 500 mg/kg, 1000 mg/kg, and 2000 mg/kg MetHbV administration groups, all cases (n = 5 in each group) survived (data not shown). In addition, in the same step as that described above, NaCN was orally administered to the mice in an amount of 12 mg/kg that was a 90% lethal dose (LD₉₀). As a result, in the 1000 mg/kg MetHbV administration group, 90% of the mice survived. However, in the 1500 mg/kg and 2000 mg/kg MetHbV administration groups that were high-concentration MetHbV administration groups, the survival rate of the mice was only 70%. Furthermore, in the 500 mg/kg MetHbV administration group, 90% of the mice died (Figure 7a). On the other hand, in the case of a combined therapy group, in which the currently clinically used existing drugs, sodium nitrite and sodium thiosulfate (NaNO₂ + Na₂S₂O₃) were administered to the mice, and a single OHCbl administration group, 90% and 50% of the mice survived, respectively (Figure 7b). From these results, it was demonstrated that MetHbV exhibits effectiveness against cyanide poisoning *in vivo,* wherein the effectiveness is equivalent to or greater than those of the existing drugs.

### 2-4-1-2. Evaluation of Effectiveness of MetHbV in Post-Treatment involving Imitated Clinical Pathological Conditions

As described in 2-4-1-1 above, the effectiveness of MetHbV in the pre-treatment was demonstrated. Thus, next, the effectiveness of MetHbV in a post-treatment for organisms suffering from cyanide poisoning was studied. Five minutes after administration of LD₉₀ NaCN, each antidote was administered to the mice, and the survival rate thereof was evaluated. As a result, in a 1000 mg/kg MetHbV administration group, and a 1000 × 2 mg/kg MetHbV administration group in which a double dose of 1000 mg/kg MetHbV was administered to the mice, all of the mice survived. In addition, also in a group in which 1000 mg/kg MetHbV and Na₂S₂O₃ (61 mg/kg) were administered to the mice in combination, all of the mice survived. On the other hand, in a combined therapy group of sodium nitrite and sodium thiosulfate (NaNO₂ + Na₂S₂O₃), 80% of the mice survived (Figure 8). Furthermore, in an OHCbl administration group, 90% of the mice survived at one hour after administration of NaCN, but when the mice were then traced until Day 7, the survival rate was decreased to 70%. Further, even in a single NaNO₂ administration group, 50% of the mice survived at one hour after administration thereof, but the survival rate was decreased to 20% until Day 7 (data not shown).

Moreover, the survival rate of the mice was evaluated also in a post-treatment in which each antidote was administered 10 minutes after administration of LD₉₀ NaCN. As a result, in the 1000 × 2 mg/kg MetHbV administration group and the combined therapy group of 1000 mg/kg MetHbV + Na₂S₂O₃ (61 mg/kg), all of the mice survived. The OHCbl administration group used as a control group had a survival rate of 60% (Figure 9). On the other hand, in the 1000 mg/kg MetHbV administration group, all of the mice survived at 1 hour after the administration thereof, but then, the survival rate was decreased to 80% until Day 7. Further, even in the single NaNO₂ administration group, the survival rate that was 30% at 1 hour after the administration was then decreased to 0% until Day 7 (data not shown).

Furthermore, the mice, which had been used to study the survival rate in the above-described post-treatment with the antidotes, were traced in terms of the time required from the intoxicated state after administration of NaCN until the recovery. Since NaCN was administered prior to each antidote, poisoning symptoms promptly appeared. Thus, 5 minutes or 10 minutes after administration of NaCN, each antidote was administered to the mice, and the time required from a coma state to return to an awakening state was then measured as an indicator of the recovery from the symptoms. As a result, in the post-treatment performed 5 minutes after NaCN administration, the mouse group regarding which the time required until the recovery was shortest was a MetHbV (1000 × 2 mg/kg) administration group (about 1 minute). In a 1000 mg/kg MetHbV administration group and a combined use group of 1000 mg/kg MetHbV + Na₂S₂O₃ (61 mg/kg), the times required until the recovery were 1.1 minutes and 2 minutes, respectively. On the other hand, in a combined therapy group of the existing drugs, sodium nitrite and sodium thiosulfate (NaNO₂ + Na₂S₂O₃), and in a single OHCbl administration group, the times required until the recovery were 2.5 minutes and 4.8 minutes, respectively (Figure 10A). Furthermore, in the post-treatment performed 10 minutes after NaCN administration, the mouse group regarding which the time required until the recovery was shortest was the same as that in the post-treatment performed 5 minutes after NaCN administration, namely, the MetHbV (1000 × 2 mg/kg) administration group (about 2 minutes). In the 1000 mg/kg MetHbV administration group and the combined use group of 1000 mg/kg MetHbV + Na₂S₂O₃ (61 mg/kg), the times required until the recovery were 3.6 minutes and 3 minutes, respectively. On the other hand, in the combined therapy group of the existing drugs, sodium nitrite and sodium thiosulfate (NaNO₂ + Na₂S₂O₃), and in the single OHCbl administration group, the times required until the recovery were 3 minutes and 4.6 minutes, respectively (Figure 10B). From these results, it was demonstrated that the use of MetHbV Contributes to the recovery of the mice from the state intoxicated with cyanide, and that the effects of the MetHbV are equivalent to or greater than those of the existing drugs.

### 2-4-2. Studies Using Hydrogen Sulfide Poisoning Mouse Models

First, in order to study the effectiveness of MetHbV in a prophylactic (pre-)treatment for hydrogen sulfide poisoning, the survival rate was evaluated. As a result, as shown in Figure 11, MetHbV was confirmed to be effective for lethal hydrogen sulfide poisoning in the prophylactic administration of the MetHbV, and the effects thereof were dose-dependent. In addition, the effects of MetHbV were higher than those of sodium nitrite or hydroxocobalamin.

Next, in order to study the effectiveness of MetHbV in a therapeutic (post-)treatment for hydrogen sulfide poisoning, the survival rate was evaluated. As a result, as shown in Figure 12, MetHbV was confirmed to have therapeutic effects against lethal hydrogen sulfide poisoning, and the effects thereof were dose-dependent. In addition, the effects of MetHbV were higher than those of sodium nitrite or hydroxocobalamin.

### 2-4-3. Studies Using Azide Poisoning Mouse Models

First, in order to study the effectiveness of MetHbV in a prophylactic (pre-)treatment for azide poisoning, the survival rate was evaluated. As a result, as shown in Figure 13, MetHbV exhibited prophylactic effects against lethal azide poisoning, and the effects thereof were dose-dependent. In addition, the effects of MetHbV were higher than those of sodium nitrite or hydroxocobalamin.

Next, in order to study the effectiveness of MetHbV in a therapeutic (post-)treatment for azide poisoning, the survival rate was evaluated. As a result, as shown in Figure 14, MetHbV was confirmed to have therapeutic effects against lethal azide poisoning, and the effects thereof were dose-dependent. In addition, the effects of MetHbV were higher than those of sodium nitrite or hydroxocobalamin.

### 2-5. Stability and Antidotal Effect of Preserved MetHbV

### 2-5-1. Stability of Preserved MetHbV

Upon utilization of MetHbV as an antidote for acute poisoning, it is desired to start the treatment with MetHbV promptly. Hence, it is important to always prepare MetHbV in advance in places, in which MetHbV is highly likely to be potentially used, such as in ambulances and in chemical plants, and this will lead to prompt use of MetHbV. For these reasons, in order to study whether MetHbV can be utilized as a "ready-to-use" highly practical antidote for acute poisoning, the long-term stability of MetHbV was evaluated under various temperature conditions. The results are shown in Table 3 and Figure 15. MetHbV that was in the state of a suspension was preserved under three temperature conditions, namely, in a cold storage state (4°C), a room temperature state (23°C to 28°C) and a warm state (37°C), for 6 months or for 1 year. As a result, the physicochemical characteristics of MetHbV after the preservation were hardly changed from fresh MetHbV, and it is considered that MetHbV is present in a stable state as a liposome even after it has been preserved for a long period of time (Table 3, Figure 15).

**[Table 3]**

| Preservation | Conditions | Diameter (nm) | ζ-potential (mV) | PDI |
|---|---|---|---|---|
| | 0 day | 219.3±4.0 | -13.2±1.0 | 0.092±0.001 |
| 4°C | 6 months | 224.9±13.4 | -13.4±2.6 | 0.075±0.026 |
| | 12 months | 212.7±3.5 | -12.9±2.2 | 0.104±0.022 |
| 23 °C | 6 months | 217.8±5.1 | -14.1±1.8 | 0.074±0.023 |
| | 12 months | 228.6±4.6 | -14.1±3.7 | 0.079±0.012 |
| 37°C | 6 months | 215.4±4.5 | -14.8±2.2 | 0.067±0.025 |
| | 12 months | 213.2±0.75 | -13.5±0.5 | 0.076±0.009 |

### 2-5-2. Antidotal Effect of Preserved MetHbV

The results obtained by studying the therapeutic effects of MetHbV preserved for a long period of time (preserved for 1 year (12 months)) against cyanide poisoning are shown in Figure 16. In lethal cyanide poisoning mouse models, all of the preserved MetHbV samples exhibited therapeutic effects (i.e. extension of the survival period) that were equivalent to those of fresh MetHbV.

Moreover, as in the case of cyanide poisoning, all of the preserved MetHbV samples exhibited therapeutic effects (i.e. extension of the survival period) that were equivalent to those of fresh MetHbV, even against hydrogen sulfide poisoning mouse models and azide poisoning mouse models (Figure 17: hydrogen sulfide poisoning, Figure 18: azide poisoning).

### Industrial Applicability

The medicament of the present embodiment has the effect of treating and preventing cyanide poisoning, hydrogen sulfide poisoning, and azide poisoning. Therefore, it is expected that the present invention will be utilized in the medical field.

## Claims

1. A medicament for cyanide poisoning, hydrogen sulfide poisoning, and azide poisoning, wherein
the medicament comprises, as an active ingredient(s), one or more selected from the group consisting of a heme protein capable of binding to cyanide ions (CN⁻), hydrogen sulfide ions (HS⁻) and azide ions (N₃⁻), a substance containing a heme protein capable of binding to CN⁻, HS⁻ and N₃⁻, and a heme derivative capable of binding to CN⁻, HS⁻ and N₃⁻.

2. The medicament according to claim 1, wherein the heme protein capable of binding to CN⁻, HS⁻ and N₃⁻ is methemoglobin.

3. The medicament according to claim 1 or 2, wherein the substance containing a heme protein capable of binding to CN⁻, HS⁻ and N₃⁻ is a methemoglobin-containing erythrocyte or a methemoglobin-containing capsule.

4. The medicament according to claim 3, wherein the capsule consists of at least one selected from the group consisting of a liposome, a polymersome, and a polymer thin film.

5. The medicament according to any one of claim1 to claim 4, which is for the treatment of cyanide poisoning, hydrogen sulfide poisoning, and azide poisoning.

6. The medicament according to any one of claim1 to claim 4, which is for the prophylaxis of cyanide poisoning, hydrogen sulfide poisoning, and azide poisoning.
